# EUROPEAN PATENT SPECIFICATION

(11) **EP 3 019 125 B1**
(45) Date of publication and mention of the grant of the patent: **17.08.2022**
(21) Application number: 14755155.0
(22) Date of filing: 11.07.2014
(51) Int. Cl.: A61F 4/00, A61F 5/01

(54) **GRIPPING AID FOR AN INDIVIDUAL HAVING A GRIPPING DEFICIENCY**
GREIFHILFE FÜR PERSONEN MIT EINGESCHRÄNKTER GREIFFUNKTION
AIDE À LA PRÉHENSION POUR UNE PERSONNE PRÉSENTANT UNE DÉFICIENCE DE PRÉHENSION

(30) Priority: 12.07.2013 IT TO20130590; 12.07.2013 IT TO20130591; 27.12.2013 IT TO20131077
(43) Date of publication of application: 18.05.2016
(73) Proprietor: Functionable S.r.l., 10129 Torino (IT)
(72) Inventor: TIRONE, Daria, 10129 Torino (IT); VENEZIANO, Federico, 10093 Collegno (IT); PERETTI, Leonardo, 10129 Torino (IT)
(74) Representative: Studio Torta S.p.A.
(86) International application number: PCT/IB2014/063046
(87) International publication number: WO 2015/004643

(56) References cited:
- GB-A- 2 476 968
- US-A- 1 217 905
- US-A- 4 447 912
- US-A- 4 606 484
- US-A- 5 542 588
- US-A- 5 853 210
- US-A1- 2010 289 282
- US-A1- 2012 285 289

## Description

### TECHNICAL FIELD

The present invention relates to a wearable portion of a gripping aid wearable by an individual suffering from a hand gripping deficiency.

### BACKGROUND ART

The hand gripping deficiency may be caused by spinal injuries that block or render inert the joints below the elbow of an upper limb, for example as a result of trauma due to road traffic accidents. As a result of these injuries, wrist and finger joints are blocked or non-reactive.

The use of a gripping aid must allow the individual to recover part of the deficiency and be as independent as possible in said functional recovery. In that regard, a preferable evaluation criteria is the simplicity with which the gripping aid is worn and adjusted. It is preferable that the individual alone is able to put on and adjust the gripping aid in complete autonomy.

Furthermore, it is important that the gripping aid allows effective locking of various tools or objects so that the individual can apply loads and perform useful operations for its own independence such as using a knife, scissors or a toothbrush.

At the same time, the gripping aid must allow the individual to adequately disconnect in a simple way the objects or tools.

As defined by the World Health Organization, with the word "aid" is intended to indicate any equipment or tool which allows a disable person to perform activities that could not otherwise be accomplished, or to carry out these activities in a safer, faster and psychologically acceptable way.

So it is possible to consider that the aid is any tool that allows a disabled person to achieve functional tasks with greater independence and safety and with less fatigue.

US1217905 discloses a hand-wearable tool for corn husking.

GB-A-2476968 discloses a golf grip training aid comprising a glove having magnetic inserts for magnetic and shape connection onto a golf club grip.

### DISCLOSURE OF INVENTION

The purpose of the present invention is to provide a wearable portion of a gripping aid capable of satisfying at least in part the requirements specified above.

The purpose of the present invention is achieved by means of a wearable portion according to claim 1 and of a gripping aid according to claim 9.

### BRIEF DESCRIPTION OF THE DRAWINGS

The invention will now be described with reference to the accompanying drawings, which illustrate a non-limiting embodiment, wherein:
- Figure 1 is a perspective view of a first component of the gripping aid according to the present invention;
- Figure 2 is a section according to a plane the trace II-II beings shown in Figure 1 both for the component of Figure 1 and a further component of the gripping aid according to the present invention;
- Figures 3 to 5 are perspective views of some objects or tools that can be coupled to the further component of the gripping aid according to the present invention; and
- Figure 6 is a schematic view illustrating additional objects or tools that can be coupled to the first component of the gripping aid.

### BEST MODE FOR CARRYING OUT THE INVENTION

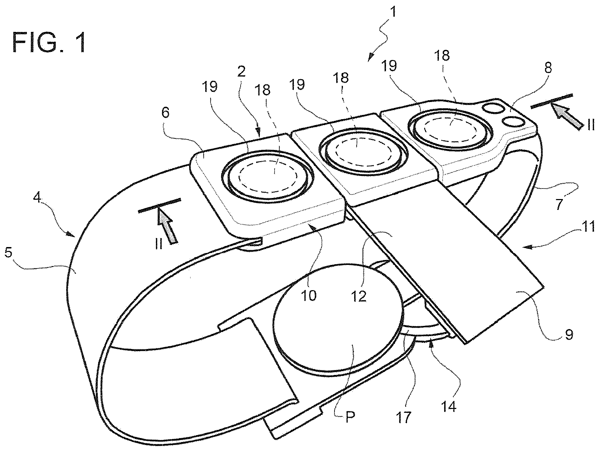

In figure 1, number 1 refers a wearable portion of a gripping aid for an individual suffering from a hand gripping deficiency which is schematically illustrated.

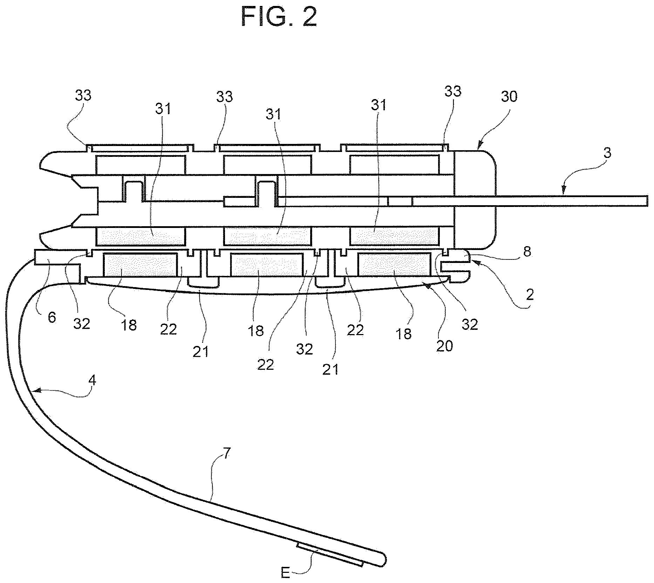

The wearable portion 1 comprises a connection member 2 connectable in a releasable manner to the detachable unit 3 of the gripping aid (Figure 2) and a band assembly 4 to fix the connection member 2 to a hand.

Preferably, the band assembly 4 comprises a first elongated member or band 5 connected to a first end 6 of the connection member 2, a second elongated member or band 7 connected to a second end portion 8 of the connection member 2 longitudinally opposite to the end portion 6, and a transverse elongated member or band 9 to connect a side 10 of the connection member arranged between the end portions 6, 8 to one of the two bands 5 and 7.

The bands 5, 7 and 9 may be of different materials and geometries one with respect to the other. Preferably, one of the two bands 5, 7 has a flexural rigidity greater than that of the other. Furthermore, the transverse band 9 is connected to the band 5 or 7 that has the greater flexural rigidity.

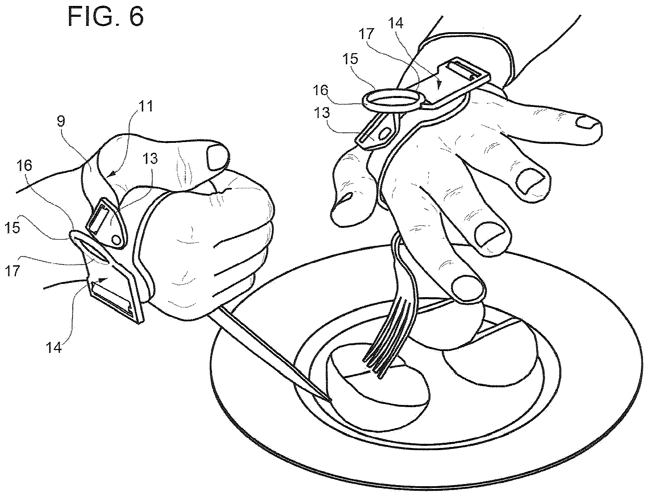

For example, the first band 5 is elastic to traction and the second band 7 is elastic to flexing and is made of a material which maintains its own shape at least under the action of gravity. Advantageously (Figure 2), the connection member 2 and the second band 7 define in cross section a 'C' shape such that, in use, the connection member 2 contacts the palm of the hand and the second band 7 surrounds a side of the hand reaching optionally also a back of the hand. Said shape is maintained in a stable manner when the connection member 2 and the second band 7 are loaded by the force of gravity. In this way, the end portions 12, 13 (Figure 6) of the transverse band 9 are maintained spaced apart along a chord of the 'C' shaped section. Furthermore, the transverse band 9, which may be similar to the first band 5 or to the second band 7, defines, with the second band 7 a passage 11 in which a thumb of the hand when the wearable portion 1 is worn can be introduced in a simple way.

The first and second band 5 and 7 are connected to each other in a releasable manner on the opposite side of the connection member 2 in use on the back or on one side of the hand. Said connection can be performed by pressure, for example, by micro-hook and micro-rings or by means of a pressure button. A male or female element E of the pressure connection is arranged on an end portion of the second band 7 as illustrated in figure 2. To connect/disconnect in a simple way the first band 5 from the second band 7, the first band 5, namely the band less rigid to flexing, comprises a ring end portion 14. The ring end portion 14 cooperates with the second band 7 to define the connection releasable by pressure preferably on the back of the hand. A ring 15 of the ring end portion 14 has a size allowing it to be engaged by a thumb or other finger and then to move the first band 5. Moreover, when the ring portion 14 is fixed to the second band 7, the ring 15 diverges from the second band 7 and has a distal end 16 opposite the first band 5 and farthest away from the back of the hand with respect to a proximal end 17 adjacent the first band 5. In this way, the ring 15 may be coupled more easily, even by using the mouth, and simplify the disconnection of the first band 5 from the second band 7. Preferably, at least the ring 15 and/or the entire portion 14 are made of plastic also to obtain an easier gripping by mouth.

The ring portion 14 may be an element shown or connected to the first band 5 and the ring 15 is not necessarily circular and can also be a hook having a shape that allows it to be engaged by a thumb or another finger.

Both the connection member 2 and the second band 7 are considered flexible, in particular elastically flexible, when a load, such as a concentrated load, is suitably applied to one of the two or to both. For this purpose, the connection member 2 and/or the second band 7 are made of a plastic material by injection or molding, even three-dimensional molding.

With reference to the connection member 2, the latter houses a plurality of magnets 18 arranged between the end portion 6 and the end portion 8. The connection member 2 defines furthermore a male or female element, in Figure 1 a female element 19, of a connection having a releasable shape.

Furthermore, according to the present invention (Figure 2), the magnets 18 are housed in a casing 20 of the connection member 2. The casing 20 is elastically flexible in the radial direction with reference to the elongated ring formed by the connection member 2, by the first and second band 5, 7. Said elastic flexing can be obtained by various expedients, among which providing weakened areas for radial deflection between two adjacent magnets 18.

Advantageously, the casing 20 defines a plurality of flexural springs 21, each of which mutually connects two adjacent magnets 18. According to a preferred embodiment of the present invention, the casing 20 has a plurality of sectors 22 substantially rigid, each of which houses a respective magnet 18. The magnets 18 are closed inside the respective sector 22 by a cover 23 which comprises, between two permanent magnets 18, at least one flexural spring 21. Preferably the cover 23 and the flexural springs 21 are made en bloc. Even more preferably, each sector 22 carries a female element 19.

Figure 2 also illustrates a section of the detachable unit 3. The detachable unit 3 includes a casing 30 housing a plurality of magnets 31 having a polarity opposite to that of the magnets 18 for generating with the latter an attraction force that keeps the detachable unit 3 and the connection member 2 in contact, and a plurality of male elements 32 that couple with the female elements 19 with a zero or minimum gap. A gap can be considered as minimum when the male element is housed in the female one and the detachable unit 3 remains in contact with the connection member 2 and can slide with respect to the latter for a maximum length less than or equal to 4 mm, preferably less than 3 mm and even more preferably less than 2 mm, along at least one direction without being detached. This direction depends on the object or tool carried by the detachable unit 3. Preferably, as illustrated in Figure 2, the male elements 32 are coupled to the female elements 19 so as to prevent the sliding of the detachable unit 3 with respect to the connection member 2 in all directions. This can for example be achieved by means of a male/female coupling with more projections on the connection member 2, or even by means of a single prismatic projection that also prevents the rotation besides the two sliding movements when the detachable unit is in contact with the connection member 2.

According to the embodiment of Figure 2, the detachable unit 3 is more rigid to flexing as compared with the connection member 2, but the connection member 2 can also be more rigid to flexing than the detachable unit 3. The difference in flexing rigidity allows to facilitate the decoupling of the magnets 31 from the magnets 18, as will be discussed below in greater detail.

Preferably, the casing 30 which houses the magnets 31 is made of a plastic material by injection molding or molding, as the connection member 2 and the second band 7.

Furthermore, the casing 30 may have a second series of male elements 33 to change the orientation of the detachable unit 3 with respect to the connection member 2. For example, as illustrated in figure 2, the male elements 33 are arranged on a side of the detachable unit 3 opposite to that of the male elements 32.

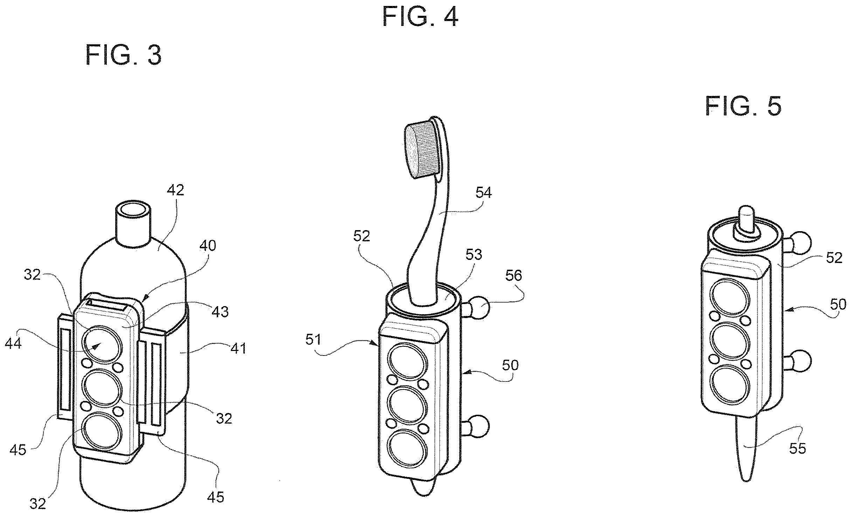

Figures 3 to 5 show further examples of a detachable unit.

In particular, a detachable unit 40 comprises an elastic band 41 and/or of adjustable length for holding an object, such as a bottle or a flask 42. Preferably, a casing 43 of the detachable unit 40 has a face 44 which bears the male elements 32 and an opposite face. The opposite face contacts the object to be held and has a shape different from that of the face 44. The casing 43 also includes appropriate buckles 45 for the anchoring and/or the adjustment of the length of the band 41. Except for as previously specified, the detachable unit 40 is equal to the detachable unit 3.

Figure 4 illustrates the detachable unit 50 in which a casing 51 comprises a tubular element 52. The tubular element 52, preferably cylindrical, comprises an adapting inner layer 53 that can be compressed around the object or tool for holding in use the latter in the tubular member 52 due to a friction action. Preferably, the tubular element 52 is opposed to the surface 44 of the male elements 32. Moreover, the adapting inner layer 53 can be of a compressible foam material fixed to an inner surface of the tubular element 52. The detachable unit 50 comprises at least a support projection 56 in the radial direction, preferably opposite to the casing 51 with respect to the tubular element 52, to stabilize its position on a flat surface and space the projections 32 from the flat surface to make the magnetic capture easier.

Figure 4 illustrates a toothbrush 54 held tight in the tubular element 52 and Figure 5 illustrates a writing instrument 55 carried by the detachable unit 50. In particular, the tubular element 52 may fit more than one tool or object on a single detachable unit. Except as previously specified, the detachable unit 50 is equal to the detachable unit 3.

The buckles 45 and the tubular element 52 are therefore multiuse connections to make the respective releasable unit 40, 50 adaptable to more objects or tools.

In use, as illustrated in figure 6, the bands 5, 7 and 9 fix the connection member 2 on the palm of the hand so that the transmission of loads and movements is effective in any direction and without the fingers acting to maintain the detachable unit 3 in contact with the connection member 2. This is due substantially to the bands 5, 7 which close around the palm and the back of the hand and the contemporary anchoring action carried out by the thumb when inserted into the passage 11 defined by the transverse band 9.

Since the releasable unit 3 carries tools to which a load will be necessarily applied, such as a knife or a fork, the magnets 18, 31 are sized to keep attached the connection member 2 and the releasable unit 3 and the shape coupling of the female elements 19 with the male elements 32 defines a barrier against the relative rotation and relative displacement movements.

In particular, the connection is made by the attraction between the magnets 18, 31 and the user can easily orient the detachable unit 3 in a suitable way.

Since the magnetic force can be elevated to withstand the loads applied in a direction parallel to the force of attraction between the magnets 18, 30, for example, each of the three magnets allows to lift a bottle of 1.5 kg, the aid should be preset so that the user can proceed in an autonomous way also to detach the detachable unit 3. To this purpose, the flexible springs 21 allow, as a result of a twisting movement of the forearm around its axis, to tilt the insert 22 with respect the casing 30 so as to space apart the magnets 18, 30. The same holds for the other magnets, so that it is possible to apply an uncoupling action for one insert 22 at a time. In this way the required load is not too high and also the uncoupling is simple.

In particular, the second band 7 has a flexural elastic rigidity greater than that of the first band 5 to allow the load to be more effectively passed onto the insert 22 proximal to the end portion 6.

Also, to further simplify the coupling/uncoupling of the detachable unit 3, the depth of the coupling between male and female elements 32, 18 is not greater than 5 mm, preferably less than 3 mm so as not to require excessive flexing of the springs 21 and too large rotations during detachment.

However, the attraction force can vary from case to case. It is in fact possible that the releasable unit 3, 40, 50, comprises only some magnets with respect to the number of magnets in the connection member 2 and/or that the magnets 31 of the releasable unit 3, 40, 50 have, in all, a magnetic field strength [Am] and/or a magnetic flux density [Tesla] lower than that of the magnets 18. In this way tools or objects that during use require relatively low loads will get a coupling/uncoupling force lower than that required for other tools or objects. This can be achieved in various ways, for example by decreasing the number and/or size and/or field strength and/or the magnetic flux density of the magnets 31 with respect to the magnets 18. Or, one or more of the magnets 31 can be fitted with reversed polarity with respect to the other magnets.

The advantages that the gripping aid according to the present invention allows to achieve are the following.

The presence of a predefined gap or the locking between the connection member 2 and 3 detachable unit allows to make more precise and reliable the use of objects or hand tools carried by the detachable unit 3.

The greater flexural rigidity of one of the two bands with respect to the other allows to keep the wearable portion 1 in an 'open' and easy to wear conformation. Furthermore, the more rigid band supports one of the portions of the releasable connection so that the closing thereof is simpler. The greater flexural rigidity of one of the two bands allows to flex the connection member 2 in a simpler way by twisting the forearm to simplify the detaching of the detachable unit 3.

Finally it is clear that modifications or variations can be made to the gripping aid here described and illustrated without departing from the scope of protection as defined by the appended claims.

It is preferable that the connection member 2 is more flexible than the detachable unit 3 since in this way the grip on the corresponding object or instrument is firmer.

The releasable bands 4 can comprise singly or in combination, straps, tubular elements, stranded ropes or similar elongated elements to fix the connection member 2 to the hand in an appropriate way.

The bands 5 and 9 are preferably made of silicone for food and medical applications to increase the long-lasting comfort and hygiene of the aid.

The projections 32 can be carried by the wearable portion 2 and the seats 19 can be carried by the detachable unit 3, 40, 50.

The seats 19 and the projections 32 do not have undercuts and can engage/disengage at least with a movement along a straight line and, in addition, with more complex movements. In combination or alternatively to this, the seats 19 and the projections 32, although adapted to be fitted on an elastically flexible support, are rigid.

The pressure connection between the first and the second band 5, 7 may be uncomfortable for the hand, made particularly sensitive and delicate by the pathological case. It is therefore preferable that the second band 7 has an end pad P (visible in Figure 1) which supports the connection member E with a large contact surface to allow the pressure on the back hand during the closure of the ring portion 14 and during the use of the gripping aid not to exceed the patient tolerability level. For this purpose, the end pad has such an extension that inscribe a circle having a diameter of at least 2 cm can be inscribed in it.

According to an embodiment of the present invention, the detachable unit 3 houses a magnetizable material, for example a ferromagnetic material and/or so-called 'soft' materials, such as the soft iron, and the connection member 2 houses a magnetic material with residual magnetization, i.e. the permanent magnets 18. In both cases, the magnetic materials are protected from the external environment by a respective casing preferably of polymeric material. In particular, each casing is watertight to allow thorough cleaning without damaging or degenerating the magnetic material due to moisture or direct contact with water. This casing is preferably obtained by molding.

Because of the presence of said casing, the coupling of the connection member 2 with the detachable unit 3 in combination with the respective shape connection is made particularly reliable and easy to detach by selecting a particular range of the energy product of the magnetic material with residual magnetization. In particular, the magnets 18 to be coupled with a ferromagnetic material housed inside the casing 31 have a product of energy measured in MegaGauss Oersteds [MGO] between 7 and 60, more preferably between 20 and 50. Moreover, the detachable unit 3, embedding a material that has no residual magnetization, can be transported and used more easily avoiding unwanted attraction forces towards other magnetic materials.

According to the present invention also those materials having a low residual magnetization but not equal to zero, in particular less than 3 MGO, more preferably less than 1 MGO (are considered functionally identical to the magnetizable or ferromagnetic materials).

## Claims

1. A wearable portion (1) of a gripping aid for a hand having a motor deficiency, comprising a releasable connection member (2) for a disconnectable unit (3) carrying an object or tool (42, 54, 55), a first elongated member (5) connected to a first end portion (6) of said connection member (2), a second elongated member (7) connected to a second end portion (8) of said connection member (2), a releasable connection between the first and second elongated member (5, 7) to close said connection member on the hand, and a third elongated member (9) arranged between a side (10) of the connection member (2) arranged between the first and the second end portion (6, 8) and one of said first and second elongated members (5, 7) to define with the latter a passage (11) which can be worn on at least one finger of the hand, the connection member (2) being in contact with the palm of the hand in use, **characterized in that** said connection member (2) is magnetic.

2. A wearable portion (1) according to claim 1, **characterized in that** said second elongated member (7) fixed to said third elongated member (9) has a higher flexural stiffness with respect to the first elongated member (5), and defines, with the connection member (2), a unit having a 'C'-shaped cross section.

3. A wearable portion according to claim 2, **characterized in that** the second elongated member (7) fixed to the third elongated member (9) is flexurally resilient.

4. A wearable portion according to claim 3, **characterized in that** the releasable connection (E, 14) comprises a gripping member (14) fixed to the first elongated member (5).

5. A wearable portion according to claim 4, **characterized in that** the gripping member (14) comprises a distal portion (16) and a proximal portion (17) with respect to said first elongated member (5), said distal portion (16) being farther from the back of the hand than the proximal portion (17) in use.

6. A wearable portion according to claim 5, **characterized in that** said gripping member (14) comprises a hook or a ring (15) configured to be hooked by a finger.

7. A wearable portion according to any one of the claims from 5 to 6, **characterized in that** the releasable connection (E, 14) is pressure connection and that the second elongated member (7) fixed to said third elongated member (9) comprises an end pad (P) which is pressed onto the back of the hand by said gripping member (14) when the releasable connection (E, 14) is closed.

8. A wearable portion according to any one of the preceding claims, **characterized in that** at least one of said first, second and third elongated members (5, 7, 9) is a band member.

9. A gripping aid for a hand having a motor deficiency comprising a wearable portion (1) according to any one of the claims from 1 to 8 and a disconnectable unit (3) for an object or hand tool (42, 54, 55) cooperating in releasable manner with the connection member (2) and a first and a second shaped connection portion (19, 32) engaging each other when the connection member (2) and the disconnectable unit (3) are maintained in contact, said first and second shaped connection portions (19, 32) defining, when reciprocally engaged, an abutment which delimits the maximum relative movement between the connection member (2) and the disconnectable unit (3) along at least a relative direction of movement.

## Patentansprüche

1. Tragbarer Abschnitt (1) einer Greifhilfe für eine Hand mit einem motorischen Defizit, umfassend ein lösbares Verbindungselement (2) für eine abtrennbare Einheit (3), die ein Objekt oder ein Werkzeug (42, 54, 55) trägt, ein erstes langgestrecktes Element (5), das mit einem ersten Endabschnitt (6) des Verbindungselements (2) verbunden ist, ein zweites langgestrecktes Element (7), das mit einem zweiten Endabschnitt (8) des Verbindungselements (2) verbunden ist, eine lösbare Verbindung zwischen dem ersten und dem zweiten langgestreckten Element (5, 7), um das Verbindungselement an der Hand zu schließen, und ein drittes langgestrecktes Element (9), das zwischen einer Seite (10) des Verbindungselements (2), die zwischen dem ersten und dem zweiten Endabschnitt (6, 8) angeordnet ist, und einem des ersten oder des zweiten langgestreckten Elements (5, 7) angeordnet ist, um mit dem Letzteren einen Durchgang (11) zu definieren, der an mindestens einem Finger der Hand getragen werden kann, wobei sich das Verbindungselement (2) bei Verwendung mit der Handfläche der Hand im Kontakt befindet, **dadurch gekennzeichnet, dass** das Verbindungselement (2) magnetisch ist.

2. Tragbarer Abschnitt (1) nach Anspruch 1, **dadurch gekennzeichnet, dass** das zweite langgestreckte Element (7), das an dem dritten langgestreckten Element (9) fixiert ist, eine höhere Biegesteifigkeit in Bezug auf das erste langgestreckte Element (5) aufweist, und mit dem Verbindungselement (2) eine Einheit definiert, die einen C-förmigen Querschnitt aufweist.

3. Tragbarer Abschnitt nach Anspruch 2, **dadurch gekennzeichnet, dass** das zweite langgestreckte Element (7), das an dem dritten langgestreckten Element (9) fixiert ist, biegeelastisch ist.

4. Tragbarer Abschnitt nach Anspruch 3, **dadurch gekennzeichnet, dass** die lösbare Verbindung (E, 14) ein Greifelement (14) umfasst, das an dem ersten langgestreckten Element (5) fixiert ist.

5. Tragbarer Abschnitt nach Anspruch 4, **dadurch gekennzeichnet, dass** das Greifelement (14) in Bezug auf das erste langgestreckte Element (5) einen distalen Abschnitt (16) und einen proximalen Abschnitt (17) umfasst, wobei in Verwendung der distale Abschnitt (16) weiter vom Rücken der Hand entfernt ist als der proximale Abschnitt (17).

6. Tragbarer Abschnitt nach Anspruch 5, **dadurch gekennzeichnet, dass** das Greifelement (14) einen Haken oder einen Ring (15) umfasst, der konfiguriert ist, um durch einen Finger eingehakt zu werden.

7. Tragbarer Abschnitt nach einem der Ansprüche 5 bis 6, **dadurch gekennzeichnet, dass** die lösbare Verbindung (E, 14) eine Druckverbindung ist und dass das zweite langgestreckte Element (7), das an dem dritten langgestreckten Element (9) fixiert ist, ein Endpolster (P) umfasst, das durch das Greifelement (14) auf den Rücken der Hand gedrückt wird, wenn die lösbare Verbindung (E, 14) geschlossen ist.

8. Tragbarer Abschnitt nach einem der vorhergehenden Ansprüche, **dadurch gekennzeichnet, dass** mindestens eines des ersten, des zweiten und des dritten langgestreckten Elements (5, 7, 9) ein Bandelement ist.

9. Greifhilfe für eine Hand mit einem motorischen Defizit, umfassend einen tragbaren Abschnitt (1) nach einem der Ansprüche 1 bis 8 und eine abtrennbare Einheit (3) für ein Objekt oder Handwerkzeug (42, 54, 55), das in lösbarer Weise mit dem Verbindungselement (2) zusammenwirkt, und einen ersten und einen zweiten geformten Verbindungsabschnitt (19, 32), die ineinander greifen, wenn das Verbindungselement (2) und die abtrennbare Einheit (3) in Kontakt gehalten werden, wobei der erste und der zweite geformte Verbindungsabschnitt (19, 32) bei gegenseitigem Eingriff einen Anschlag definieren, der die maximale relative Bewegung zwischen dem Verbindungselement (2) und der abtrennbaren Einheit (3) entlang zumindest einer relativen Bewegungsrichtung begrenzt.

## Revendications

1. Partie portable (1) d'une aide à la préhension pour une main présentant une déficience motrice, comprenant un élément de connexion (2) libérable pour une unité déconnectable (3) portant un objet ou outil (42, 54, 55), un premier élément allongé (5) connecté à une première partie d'extrémité (6) dudit élément de connexion (2), un deuxième élément allongé (7) connecté à une deuxième partie d'extrémité (8) dudit élément de connexion (2), une connexion libérable entre le premier et deuxième élément allongé (5, 7) pour fermer ledit élément de connexion d'une part, et un troisième élément allongé (9) agencé entre un côté (10) de l'élément de connexion (2) agencé entre la première et la deuxième partie d'extrémité (6, 8) et un desdits premier et deuxième éléments allongés (5, 7) pour définir avec ceuxci un passage (11) qui peut être porté sur au moins un doigt de la main, l'élément de connexion (2) étant en contact avec la paume de la main lors d'une utilisation, **caractérisée en ce que** ledit élément de connexion (2) est magnétique.

2. Partie portable (1) selon la revendication 1, **caractérisée en ce que** ledit deuxième élément allongé (7) fixé audit troisième élément allongé (9) présente une rigidité en flexion plus élevée par rapport au premier élément allongé (5), et définit, avec l'élément de connexion (2), une unité présentant une section transversale en forme de « C ».

3. Partie portable selon la revendication 2, **caractérisée en ce que** le deuxième élément allongé (7) fixé au troisième élément allongé (9) est élastique en flexion.

4. Partie portable selon la revendication 3, **caractérisée en ce que** la connexion libérable (E, 14) comprend un élément de préhension (14) fixé au premier élément allongé (5).

5. Partie portable selon la revendication 4, **caractérisée en ce que** l'élément de préhension (14) comprend une partie distale (16) et une partie proximale (17) par rapport audit premier élément allongé (5), ladite partie distale (16) étant plus éloignée du dos de la main que la partie proximale (17) lors d'une utilisation.

6. Partie portable selon la revendication 5, **caractérisée en ce que** ledit élément de préhension (14) comprend un crochet ou un anneau (15) configuré pour s'accrocher à un doigt.

7. Partie portable selon l'une quelconque des revendications 5 à 6, **caractérisée en ce que** la connexion libérable (E, 14) est une connexion à pression et que le deuxième élément allongé (7) fixé audit troisième élément allongé (9) comprend un patin d'extrémité (P) qui est pressé sur le dos de la main par ledit élément de préhension (14) lorsque la connexion libérable (E, 14) est fermée.

8. Partie portable selon l'une quelconque des revendications précédentes, **caractérisée en ce qu'**au moins un desdits premier, deuxième et troisième éléments allongés (5, 7, 9) est un élément formant bande.

9. Aide à la préhension pour une main présentant une déficience motrice comprenant une partie portable (1) selon l'une quelconque des revendications 1 à 8 et une unité déconnectable (3) pour un objet ou outil à main (42, 54, 55) coopérant d'une manière libérable avec l'élément de connexion (2) et une première et une deuxième partie de connexion façonnée (19, 32) en prise l'une avec l'autre lorsque l'élément de connexion (2) et l'unité déconnectable (3) sont maintenus en contact, lesdites première et deuxième parties de connexion façonnées (19, 32) définissant, lorsqu'elles sont réciproquement en prise, une butée qui délimite le mouvement relatif maximum entre l'élément de connexion (2) et l'unité déconnectable (3) le long d'au moins une direction de mouvement relative.
